# EUROPÄISCHE PATENTANMELDUNG

(11) **EP 3 384 828 A1**
(43) Veröffentlichungstag der Anmeldung: **10.10.2018**
(21) Anmeldenummer: 17164780.3
(22) Anmeldetag: 04.04.2017
(51) Int. Cl.: A61B 5/00, G06F 19/00

(54) **MAKROSKOPISCHES ÜBERWACHEN DES GESUNDHEITSSTATUS EINES KOLLEKTIVS**

(71) Anmelder: Siemens Healthcare GmbH, 91052 Erlangen (DE)
(72) Erfinder: SCHMIDT, Sebastian, 91085 Weisendorf (DE); IONASEC, Razvan, 90402 Nuernberg (DE); SHAH, Amitkumar Bhupendrakumar, 91052 Erlangen (DE)

(57) **Zusammenfassung**

Es wird ein Verfahren zum Überwachen des Gesundheitsstatus eines Kollektivs beschrieben. Bei dem Verfahren werden medizinische Bilddatensätze (BDₙₖ) einer Mehrzahl von Mitgliedern des Kollektivs erfasst. Es werden Mitglieder (Pₙ) des Kollektivs auf Basis der erfassten medizinischen Bilddatensätze (BDₙₖ) nach einem medizinisch relevanten Kriterium automatisiert klassifiziert. Dann erfolgt ein automatisiertes Ermitteln einer statistischen Verteilung (SV_{R}) der klassifizierten Mitglieder (Pₙ) des Kollektivs. Schließlich erfolgt ein automatisiertes Vergleichen einer statistischen Referenz-Verteilung mit der ermittelten statistischen Verteilung (SV_{R}). Es wird auch eine Überwachungseinrichtung (30) beschrieben.

## Beschreibung

Die Erfindung betrifft ein Verfahren zum Überwachen des Gesundheitsstatus eines Kollektivs. Weiterhin betrifft die Erfindung eine Überwachungseinrichtung.

Bei der Behandlung von sehr häufig auftretenden ernsthaften Erkrankungen, wie zum Beispiel epidemisch auftretenden Infektionskrankheiten, ist eine überregionale Überwachung der Ausbrüche und der Behandlung dieser Krankheiten sinnvoll, um zentral gesteuert Gegenmaßnahmen einleiten zu können.

Häufig eingesetzte Arzneimittel zur Behandlung von bakteriellen Erkrankungen sind Antibiotika. Bei einem großflächigen Einsatz solcher Antibiotika treten Resistenzen auf, die eine große Herausforderung in der modernen Medizin sind. Bakterien ändern ihren Gencode, um Resistenzen gegen spezifische Antibiotika zu entwickeln, die dann keinen therapeutischen Effekt mehr zeigen. Dadurch kann der Patient nicht mehr effektiv behandelt werden und es geht wertvolle Zeit verloren, um eine effektive Therapie zu finden bzw. durchzuführen. Weiterhin geht viel Geld für wirkungslose Medikamente verloren. Beispielsweise hat Mycobacterium tuberculosis, welches Tuberkulose verursacht, eine Resistenz gegen multiple Antibiotika (MDR-TB) in einem großen Teil der Bevölkerung Südasiens entwickelt. Infolge der Unwirksamkeit von Tuberkulosemedikamenten sind die Überlebenschancen der Patienten stark gesunken.

Informationen über die geographische Verteilung von resistenten Bakterienstämmen sind notwendig, um geeignete Empfehlungen für die Verwendung von Antibiotika und andere Maßnahmen abzugeben. Beispielsweise ist es möglich, Patienten in bestimmten Gebieten zu isolieren, die eine hohe Prävalenz für eine Resistenz gegen Antibiotika haben oder sie in spezialisierte Krankenhäuser oder Pflegeeinrichtung zu verbringen.

Lokale Behandlungsinformationen können dazu genutzt werden, um die Situation in betroffenen Gebieten sowohl für stationäre Patienten als auch für ambulante Patienten zu beherrschen.

Ebenso ist es nützlich, die lokale Verteilung von Krankheiten einschließlich weiterer Infektionen, beruflicher Risiken und Umweltrisiken zu kennen. Zum Beispiel erhöht sich die Anzahl von Virusinfektionen üblicher Weise in den Wintermonaten. Solche Infektionen breiten sich regional aus und können Maßnahmen, wie zum Beispiel Massenimpfungen oder eine Intensivierung der medizinischen Versorgung, erfordern. Andere Anwendungsgebiete sind Epidemien, wie das Zikavirus oder Krankheiten, welche durch externe Faktoren bedingt sind, wie zum Beispiel das Pleuramesotheliom, welches durch Asbest verursacht wird.

Leider sind Resistenzen von Antibiotika heutzutage nur durch komplexe Labortests ermittelbar, wobei Proben vom Patienten genommen werden. Es ist schwierig, diese Tests für eine größere Anzahl von Patienten auszuwerten, um zum Beispiel einen schnellen Überblick über die Situation in einer großen Stadt zu erhalten. Daher wird ein Verfahren benötigt, mit dem automatisiert Daten in großem Umfang analysiert werden können und ein Überblick über große Populationen erhalten werden kann.

Für Grippeinfektionen in Deutschland gibt es ein Netzwerk von Arztpraxen, welche alle Infektionen an das Robert Koch Institut übermitteln. Allerdings bedeutet dies einen erheblichen Aufwand und eine solche Vorgehensweise lässt sich nur bei bestimmten Krankheiten durchführen.

Für das Auftreten von Krebs gibt es Register in vielen Staaten, wie zum Beispiel das SEER in den USA. Allerdings sind diese oft unvollständig und Daten werden langsam übermittelt, so dass schnelle Reaktionen nicht möglich sind.

Bildgebende Verfahren, wie zum Beispiel die optische Mikroskopie, die Fluoreszenz, Röntgenstrahlen, Ultraschall, CT, MRT, PET, SPECT usw., werden routinemäßig während klinischer Untersuchungen von Medikamenten genutzt, um die Wirksamkeit bestimmter Medikamente gegen eine bestimmte Krankheit nachzuweisen. Bei diesen Untersuchungen werden die Patienten vor der Behandlung und eventuell mehrere Male während der Zeit der Behandlung und nach der Behandlung mit einem bildgebenden Verfahren untersucht. Bei jeder Bildaufnahme werden Biomarker ermittelt und verglichen, um die Reaktion des Patienten auf eine Behandlung zu ermitteln.

In vielen Gebieten werden Verfahren mit Lernalgorithmen, wie zum Beispiel "deep learning" oder "machine learning" angewandt. Eine große Anzahl von Datensätzen mit bekannten Informationen werden verwendet, um eine Software zu trainieren, die dann dazu in der Lage ist, vorher unbekannte Datensätze zu analysieren. Diese Verfahren werden aktuell breit angewandt für Aufgaben in der Bildanalyse, wie zum Beispiel Personenerkennung oder bei selbstfahrenden Fahrzeugen. Bei medizinischen Diagnoseverfahren jedoch sind die Ergebnisse oft nicht exakt genug, um eine Entscheidung für einen Patienten zu treffen.

Es besteht also das Problem, ein Verfahren zum Überwachen des Gesundheitsstatus in einem großen Kollektiv zu entwickeln, welches mit geringerem Aufwand als bei den herkömmlichen Verfahren durchgeführt werden kann.

Diese Aufgabe wird durch ein Verfahren zum Überwachen des Gesundheitsstatus eines Kollektivs gemäß Patentanspruch 1 und durch eine Überwachungseinrichtung gemäß Patentanspruch 13 gelöst.

Bei dem erfindungsgemäßen Verfahren zum Überwachen des Gesundheitsstatus eines Kollektivs werden medizinische Bilddatensätze einer Mehrzahl von Mitgliedern des Kollektivs erfasst. Als Gesundheitsstatus soll in der Anmeldung eine Beschreibung und oder Messgröße der Gesundheit eines Individuums oder eines größeren Kollektivs, beispielsweise einer Bevölkerung, zu einem bestimmten Zeitpunkt im Hinblick auf identifizierbare Standards, gewöhnlich mit Hilfe von Gesundheitsindikatoren verstanden werden. Die medizinischen Bilddatensätze können zum Beispiel in einer zentralen medizinischen Datenbank gespeichert sein und von dieser abgerufen werden. Die medizinischen Bilddatensätze können jedoch auch zunächst dezentral auf einzelnen bildgebenden Einrichtungen zugeordneten Bilddatenspeichern verteilt abgespeichert sein. Die medizinischen Bilddatensätze umfassen Bilddaten, welche als Messdaten bzw. Messwerte von den jeweiligen Mitgliedern des Kollektivs zu verstehen sind. Weiterhin erfolgt ein automatisiertes Klassifizieren der Mitglieder des Kollektivs auf Basis der erfassten medizinischen Bilddatensätze nach einem medizinisch relevanten Kriterium. Ein solches medizinisch relevantes Kriterium kann sich zum Beispiel auf gesundheitsbezogene Fragestellungen beziehen, vorzugsweise auf den jeweiligen Gesundheitsstatus der Mitglieder des Kollektivs, wobei die Klassifizierung vor dem Hintergrund einer solchen Fragestellung vorgenommen wird. Der Klassifizierungsschritt kann zentral nach Empfangen der medizinischen Bilddaten von einer zentralen Datenbank erfolgen, er kann aber auch verteilt, d.h. unmittelbar an den einzelnen Standorten vorgenommen werden, an denen die einzelnen Mitglieder des Kollektivs medizinisch betreut werden. Die Klassifizierung kann kontinuierlich erfolgen, d.h. jeweils dann, wenn ein neuer medizinischer Bilddatensatz von einem Mitglied erfasst wird, wird das Mitglied des Kollektivs entsprechend klassifiziert. Alternativ kann die Klassifizierung auch nur zu vorbestimmten festen Zeitpunkten auf Basis der zu diesem Zeitpunkt zur Verfügung stehenden medizinischen Bilddaten erfolgen.

Nachfolgend erfolgt ein automatisiertes Ermitteln einer statistischen Verteilung der klassifizierten Mitglieder des Kollektivs. Dieser Vorgang sollte im Gegensatz zu dem Klassifizierungsschritt zentral erfolgen, denn zur Ermittlung der Statistik werden üblicherweise klassifizierte Mitglieder des Kollektivs von verschiedenen Standorten bzw. Betreuungseinrichtungen berücksichtigt. Schließlich wird eine statistische Referenz-Verteilung mit der ermittelten statistischen Verteilung verglichen. Als statistische Verteilung der klassifizierten Mitglieder des Kollektivs soll eine Verteilung der Mitglieder des Kollektivs über die bei der Klassifizierung verwendeten Klassen verstanden werden. Dabei kann eine Klasseneinteilung gegebenenfalls auch kontinuierlich erfolgen. Die statistische Verteilung kann auch von weiteren Größen, wie zum Beispiel vom Ort oder der ethnischen Zugehörigkeit usw. abhängen. Der statistischen Referenz-Verteilung ist ein im Zusammenhang mit der Fragestellung stehender wohldefinierter Zustand des Kollektivs zugeordnet. Durch den Vergleich kann auf die tatsächliche innerhalb des Kollektivs vorherrschende Situation in Bezug auf die genannte Fragestellung geschlossen werden. Beispielsweise kann ab einer vorbestimmten Abweichung der ermittelten statistischen Verteilung von der Referenz-Verteilung davon ausgegangen werden, dass eine stimmte Problematik bei der Behandlung zumindest eines Teils des Kollektivs vorliegt, gegen die Gegenmaßnahmen erfolgen sollten. Vorteilhaft können die Schritte des Verfahrens automatisiert ablaufen, so dass kein Fachpersonal zur Auswertung der Bilddatensätze benötigt wird. Da bei der Analyse nur die Gesamtsituation des Kollektivs im Vordergrund steht, können Fehler bei der individuellen Klassifikation einzelner Mitglieder des Kollektivs toleriert werden.

Die erfindungsgemäße Überwachungseinrichtung weist eine Eingangsschnittstelle zum Erfassen von Bilddatensätzen einer Mehrzahl von Mitgliedern eines Kollektivs auf. Teil der erfindungsgemäßen Überwachungseinrichtung ist auch eine Klassifizierungseinheit zum automatisierten Klassifizieren der Mitglieder des Kollektivs auf Basis der erfassten Bilddatensätze nach einem medizinisch relevanten Kriterium. Die erfindungsgemäße Überwachungseinrichtung umfasst zudem eine Verteilungsermittlungseinheit zum automatisierten Ermitteln einer statistischen Verteilung der klassifizierten Mitglieder des Kollektivs und eine Vergleichseinheit zum automatisierten Vergleichen einer statistischen Referenzverteilung mit der ermittelten statistischen Verteilung.

Die wesentlichen Komponenten der erfindungsgemäßen Überwachungseinrichtung können zum überwiegenden Teil in Form von Softwarekomponenten ausgebildet sein. Dies betrifft insbesondere Teile der Klassifizierungseinheit, der Verteilungsermittlungseinheit und der Vergleichseinheit. Grundsätzlich können diese Komponenten aber auch zum Teil, insbesondere wenn es um besonders schnelle Berechnungen geht, in Form von softwareunterstützter Hardware, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden.

Eine weitgehend softwaremäßige Realisierung hat den Vorteil, dass auch schon bisher für medizinische Aufgaben genutzte verwendete Rechnersysteme auf einfache Weise durch ein Software-Update nachgerüstet werden können, um auf die erfindungsgemäße Weise als Überwachungseinrichtung zu arbeiten. Insofern wird die Aufgabe auch durch ein entsprechendes Computerprogrammprodukt mit einem Computerprogramm gelöst, welches direkt in eine Speichereinrichtung eines solchen, als Überwachungseinrichtung genutzten Rechnersystems ladbar ist, mit Programmabschnitten, um alle Schritte des erfindungsgemäßen Verfahrens auszuführen, wenn das Computerprogramm in dem Rechnersystem ausgeführt wird.

Ein solches Computerprogrammprodukt kann neben dem Computerprogramm gegebenenfalls zusätzliche Bestandteile wie z.B. eine Dokumentation und/oder zusätzliche Komponenten auch Hardware-Komponenten, wie z.B. Hardware-Schlüssel (Dongles etc.) zur Nutzung der Software, umfassen
Zum Transport zur Speichereinrichtung des Rechnersystems und/oder zur Speicherung an dem Rechnersystem kann ein computerlesbares Medium, beispielsweise ein Memorystick, eine Festplatte oder ein sonstiger transportabler oder fest eingebauter Datenträger dienen, auf welchem die von einem Rechnersystem einlesbaren und ausführbaren Programmabschnitte des Computerprogramms gespeichert sind. Das Rechnersystem kann z.B. hierzu einen oder mehrere zusammenarbeitende Mikroprozessoren oder dergleichen aufweisen.

Die abhängigen Ansprüche sowie die nachfolgende Beschreibung enthalten jeweils besonders vorteilhafte Ausgestaltungen und Weiterbildungen der Erfindung. Dabei können insbesondere die Ansprüche einer Anspruchskategorie auch analog zu den abhängigen Ansprüchen einer anderen Anspruchskategorie und deren Beschreibungsteilen weitergebildet sein. Zudem können im Rahmen der Erfindung auch die verschiedenen Merkmale unterschiedlicher Ausführungsbeispiele und Ansprüche auch zu neuen Ausführungsbeispielen kombiniert werden.

In einer Ausgestaltung des erfindungsgemäßen Verfahrens zum Überwachen des Gesundheitsstatus eines Kollektivs werden bei dem Klassifizierungsschritt den einzelnen Mitgliedern zugeordnete medizinische Parameter auf Basis der erfassten Bilddatensätze ermittelt und das automatisierte Klassifizieren der Mitglieder des Kollektivs erfolgt auf Basis der ermittelten medizinischen Parameter. Die genannten medizinischen Parameter umfassen quantitative Informationen bezüglich medizinisch relevanter Vorgänge und Zustände im Körper eines Patienten. Die genannten medizinischen Parameter beruhen auf einer Analyse der erfassten Bilddatensätze, deren Daten als Messwerte zu verstehen sind. Ein Beispiel für solche medizinischen Parameter sind sogenannte Biomarker. Biomarker umfassen charakteristische biologische Merkmale, die objektiv gemessen werden können und auf einen normalen biologischen oder krankhaften Prozess im Körper hinweisen können. Bei einem Biomarker kann es sich um makroskopisch erkennbare Veränderungen bzw. Auffälligkeiten in einem zu untersuchten Körper handeln, es kann sich auch um mikroskopisch oder mit Hilfe von Analyseverfahren zu identifizierende bzw. zu untersuchende biologische Materialien, wie zum Beispiel Zellen, Gene, Genprodukte oder bestimmte Moleküle wie Enzyme oder Hormone, handeln. Auch komplexe Organfunktionen oder charakteristische Veränderungen biologischer Strukturen werden als medizinische Biomarker herangezogen. Vorteilhaft werden bei dem erfindungsgemäßen Überwachungsverfahren die Biomarker automatisiert ermittelt.

Ein solches automatisiertes Klassifizieren der Mitglieder des Kollektivs kann durch Anwendung eines trainingsbasierten Klassifizierungsverfahrens erfolgen. Besonders vorteilhaft kann ein solches trainingsbasiertes Verfahren an das Erkennen von spezifischen Arten von Biomarkern individuell angepasst werden, so dass das Verfahren sehr breit und flexibel angewendet werden kann.

In einer Ausgestaltung des erfindungsgemäßen Verfahrens zum Überwachen des Gesundheitsstatus eines Kollektivs werden Steuermaßnahmen zur Beeinflussung des Gesundheitsstatus des Kollektivs auf Basis eines Ergebnisses des Vergleichs vorzugsweise automatisiert eingeleitet. Solche Maßnahmen können zum Beispiel Warnungen oder Anweisungen zur Durchführung bestimmter Korrektur- oder Schutzmaßnahmen gezielt gegenüber Teilen des überwachten Kollektivs umfassen. Vorteilhaft kann eine medizinische Betreuung zentral überwacht und gesteuert werden. Das Einleiten von medizinisch wirksamen Maßnahmen kann zum Beispiel für den Fall erfolgen, dass die ermittelte statistische Verteilung von der statistischen Referenz-Verteilung abweicht. Ein solches Abweichen kann in diesem Fall auf eine mangelhafte Wirksamkeit oder gar Schädlichkeit einer bisherigen medizinischen Betreuung hinweisen und dazu genutzt werden, die Betreuung eines Teils des Patientenkollektivs zu modifizieren.

In einer besonders bevorzugten Variante des erfindungsgemäßen Verfahrens zum Überwachen des Gesundheitsstatus eines Kollektivs erfolgt das automatisierte Klassifizieren auf Basis von Zeitreihen von Bilddatensätzen. Bei dieser Variante wird eine Zeitabhängigkeit von auf Basis der medizinischen Bilddaten analysierten medizinisch relevanten Größen untersucht, um die einzelnen Mitglieder des Kollektivs zu klassifizieren. Vorteilhaft können bei einer solchen Vorgehensweise Aspekte eines Krankheitsverlaufs oder den Erfolg oder Misserfolg einer Behandlung eines Patienten kennzeichnende zeitabhängige Größen als Kriterium für eine Klassifizierung verwendet werden.

In einer bevorzugten Ausgestaltung des erfindungsgemäßen Verfahrens zum Überwachen des Gesundheitsstatus eines Kollektivs wird das trainingsbasierte Klassifizierungsverfahren dadurch trainiert, dass ein initiales Klassifizierungsverfahren auf eine Mehrzahl von Bilddatensätzen mit vorbekannter Klassifizierung der den Bilddatensätzen zugeordneten Mitgliedern des Kollektivs, beispielsweise Patienten oder auch gesunde Personen, angewandt wird. Weiterhin wird im Rahmen des Trainingsprozesses das Klassifizierungsergebnis mit einem vorbekannten Klassifizierungsergebnis der Mehrzahl von Bilddatensätzen verglichen. Nachfolgend erfolgt ein Anpassen des initialen Klassifizierungsverfahrens auf Basis des Vergleichs. Die vorgenannten Schritte werden mit dem angepassten Klassifizierungsverfahren wiederholt und der skizzierte Anpassvorgang wird so oft iterativ fortgesetzt, bis ein Qualitätskriterium für das angepasste Ermittlungsverfahren erreicht ist. Schließlich wird das dem Qualitätskriterium entsprechende angepasste Klassifizierungsverfahren als Klassifizierungsverfahren für das erfindungsgemäße Verfahren festgelegt. Vorteilhaft wird das Klassifizierungsverfahren auf eine bestimmte Art von Fragestellung und gegebenenfalls auf einen bestimmten Patientenkreis angepasst, so dass die Ergebnisse der Klassifizierung besonders genau ermittelt werden können.

In einer Ausgestaltung des erfindungsgemäßen Verfahrens zum Überwachen des Gesundheitsstatus eines Kollektivs wird das trainingsbasierte Klassifizierungsverfahren mit Hilfe eines maschinellen Lernverfahrens trainiert. Ein solches Lernverfahren kann zum Beispiel unter Anwendung eines künstlichen neuronalen Netzes realisiert werden. Mit den beschriebenen Verfahren wird die Anpassung des Klassifizierungsverfahrens an eine bestimmte medizinische Fragestellung oder an ein bestimmtes Kollektiv automatisiert, so dass der Personalaufwand für die Überwachung des Kollektivs reduziert werden kann bzw. eine solche Überwachung in Ländern mit niedrigem formalen Bildungsstand erst möglich gemacht werden kann.

In einer besonders bevorzugten Ausgestaltung des erfindungsgemäßen Verfahrens zum Überwachen des Gesundheitsstatus eines Kollektivs umfasst die statistische Verteilung der klassifizierten Mitglieder des Kollektivs eine geographische statistische Verteilung. Beispielsweise werden bei einer solchen statistischen Verteilung Anteile von in bestimmte Klassen eingeteilte Patienten an der Gesamtzahl der Patienten am jeweiligen Ort in Abhängigkeit vom Ort ermittelt. Vorteilhaft können Änderungen bei der Betreuung von Patienten auf geographisch beschränkte Gebiete konzentriert werden, wodurch die Zielgenauigkeit und Effektivität der Maßnahmen verbessert wird.

Das automatisierte Klassifizieren kann zusätzlich zu den erfassten Bilddatensätzen auch auf Basis von den einzelnen Bilddatensätzen zugeordneten Zusatzinformationen erfolgen. Die genannten Zusatzinformationen können beispielsweise einen oder mehrere der folgenden Informationstypen umfassen:
- Diagnosedaten,
- Laborberichte,
- Datum und Zeitpunkt der Bildaufnahme der Bilddatensätze. Die genannten Zusatzinformationen können auch auf anderem Wege ermittelt Biomarker umfassen. Beispielsweise können an den überwachten Personen Untersuchungen auf Krankheitserreger oder sonstige pathologische Phänomene durchgeführt worden sein und den erfassten Bilddatensätzen beigefügt sein.

In einer besonders sinnvoll anwendbaren Variante des erfindungsgemäßen Verfahrens zum Überwachen des Gesundheitsstatus eines Kollektivs wird auf Basis des Vergleichs eine Prävalenz des Kollektivs bezüglich einer vorbestimmten Krankheit oder ein Ansprechen des Kollektivs auf ein Medikament ermittelt. Vorteilhaft können mit Hilfe des erfindungsgemäßen Verfahrens Behandlungsmaßnahmen oder die Verteilung von Medikamenten an in dem Kollektiv vorherrschende besondere Umstände angepasst werden.

Die Erfindung wird im Folgenden unter Hinweis auf die beigefügten Figuren anhand von Ausführungsbeispielen noch einmal näher erläutert. Es zeigen:
FIG 1 ein Flussdiagramm, welches ein Verfahren zum Überwachen eines Gesundheitsstatus in einem Kollektiv veranschaulicht,
FIG 2 ein Flussdiagramm, welches einen Trainingsvorgang zur Anpassung eines trainingsbasierten Ermittlungsverfahrens veranschaulicht,
FIG 3 ein Blockdiagramm, mit dem eine Überwachungseinrichtung gemäß einem Ausführungsbeispiel der Erfindung dargestellt wird.

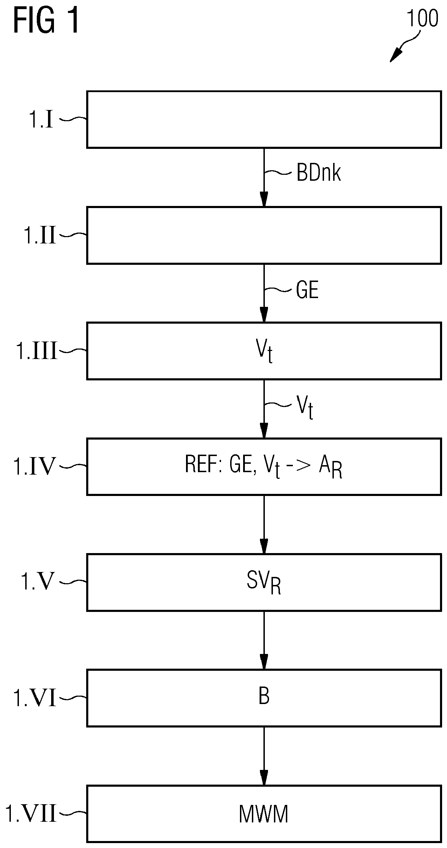

In FIG 1 ist ein Flussdiagramm 100 gezeigt, welches ein Verfahren zum Überwachen eines Gesundheitsstatus in einem Kollektiv gemäß einem Ausführungsbeispiel der Erfindung veranschaulicht. Bei dem in FIG 1 veranschaulichten Ausführungsbeispiel werden Fälle von Lungenentzündungen in einem großen Gebiet, beispielsweise einem ganzen Staatsgebiet überwacht. Hierfür werden bei dem Schritt 1.1 zunächst Serien von jeweils K Bilddatensätzen einer Anzahl N von Patienten aus einer zentralen Datenbank bezogen, in der diese zentral gespeichert sind, wobei K und N natürliche Zahlen sind. Die insgesamt N * K Bilddatensätze wurden in verschiedenen Behandlungseinrichtungen, beispielsweise Krankenhäuser oder Arztpraxen, zu verschiedenen Zeitpunkten in verschiedenen Behandlungsstadien aufgenommen. Beispielsweise wurde von einem n-ten Patient ein erster Bilddatensatz BDₙ₁ vor dem Beginn der Behandlung des Patienten aufgenommen. Anschließend wurden Bilddatensätze BDₙ₂, BDₙ₃ ... BD_{nK} während des Verlaufs der Behandlung von dem Patienten aufgenommen, um das Ansprechen des Patienten auf die Behandlung zu dokumentieren.

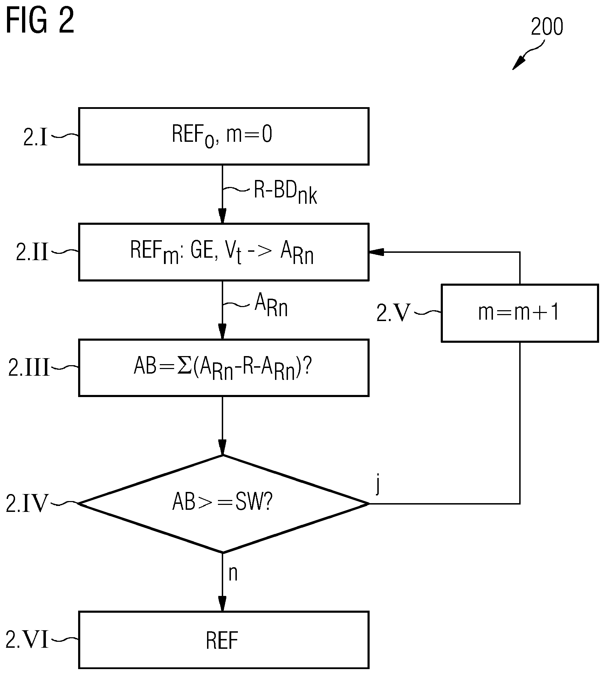

In dem in FIG 1 gezeigten konkreten Ausführungsbeispiel erfolgt eine Behandlung der Patienten mit Hilfe eines Antibiotikums. Bei dem Schritt 1.II werden dann anhand der Bilddatensätze BDₙₖ jeweils die Größenabmessungen GE der entzündeten Bereiche der Lunge ermittelt. Dieser Biomarker GE wird bei dem Schritt 1.III dazu genutzt, anhand einer Serie von Bildern BDₙ₁, BDₙ₂, BDₙ₃ eines jeweiligen Patienten Pₙ einen Krankheitsverlauf Vₜ in Abhängigkeit von der Zeit t zu ermitteln. Dieser Ermittlungsprozess wird für jeden Patienten Pₙ durchgeführt. Bei dem Schritt 1.IV wird dann auf Basis des Krankheitsverlaufs Vₜ ermittelt, ob ein jeweiliger Patient Pₙ auf das Antibiotikum anspricht oder ob sich Resistenzen gegen das Antibiotikum entwickelt haben. Dabei wird ein logischer Wert A_{R} ermittelt. Liegt der Wert A_{R} bei 1, so liegt eine Resistenz vor, liegt der Wert A_{R} bei 0, so liegt keine Resistenz vor. Das Ermitteln kann auf Basis eines trainierten Ermittlungsverfahrens bzw. mit Hilfe einer trainierten Resistenz-Ermittlungsfunktion REF erfolgen, wie es in dem in FIG 2 gezeigten Flussdiagramm 200 veranschaulicht ist. Zwar können einzelne Resistenzwerte A_{R} falsch sein, jedoch ist davon auszugehen, dass diese Fehler bei einem entsprechend gut trainierten Ermittlungsverfahren bei einer sehr großen Gesamtheit von Patienten nicht ins Gewicht fallen.

Bei dem Schritt 1.V wird anhand der ermittelten Resistenzwerte A_{R} eine Verteilung SV_{R} von Resistenzen über das gesamte überwachte Gebiet ermittelt. Weiterhin werden bei dem Schritt 1.VI auf Basis der ermittelten Verteilung SV_{R} Gebiete B ermittelt, in denen eine Anwendung des bisher verwendeten Antibiotikums aufgrund einer zu hohen Anzahl oder Dichte des Auftretens von Resistenzen zukünftig unterbleiben soll. Diese Entscheidung kann zum Beispiel durch Vergleich der ermittelten Verteilung SV_{R} mit einer Referenz-Verteilung bzw. einem Referenzwert erfolgen, der zum Beispiel einen Schwellwert umfasst, der eine gerade noch akzeptable Verteilungsdichte von Resistenzfällen beschreibt. Bei dem Schritt 1.VII werden dann Anweisungen MWM an Kliniken und andere Behandlungszentren in den ermittelten Gebieten B gegeben, dass die Therapie der Patienten entsprechend geändert werden soll. Es soll an dieser Stelle erwähnt werden, dass insbesondere die Schritte 1.1 bis 1.VI vorzugsweise automatisiert erfolgen, so dass der personelle Aufwand für eine Durchführung des beschriebenen Verfahrens gering gehalten werden kann.

In FIG 2 ist ein Flussdiagramm 200 gezeigt, welches einen Trainingsvorgang zur Anpassung eines trainingsbasierten Ermittlungsverfahrens veranschaulicht. Bei dem in FIG 2 gezeigten Verfahren wird eine Resistenz-Ermittlungsfunktion REF mit Hilfe von Referenzbilddaten R-BDₙₖ dazu trainiert, Resistenzen A_{Rn} von den Referenzbilddaten R-BDₙₖ zugeordneten Referenz-Personen R-Pₙ auf Basis einer Bildanalyse korrekt zu ermitteln. Bei dem Schritt 2.1 wird zunächst eine initiale Resistenz-Ermittlungsfunktion REF₀ festgelegt, welche beispielsweise auf Basis einer Modellüberlegung Resistenzen A_{Rn} auf Basis von Referenz-Bilddatensätzen R-BDₙₖ ermittelt. Bei dem Schritt 2.II wird die initiale Resistenz-Ermittlungsfunktion REF₀ dann auf die Referenz-Bilddatensätze R-BDₙₖ angewandt und es werden Resistenzwerte A_{Rn} auf Basis der Referenz-Bilddatensätze R-BDₙₖ ermittelt.

Bei dem Schritt 2.111 wird ein Maß für eine Abweichung AB der Gesamtheit der Resistenzwerte A_{Rn}, welche mit Hilfe des initialen Ermittlungsverfahrens für die Referenz-Bilddatensätze R-BDₙₖ ermittelt wurden, von einem vorbekannten Datensatz von Referenz-Resistenzwerten R-A_{Rn}, welche den Referenz-Bilddatensätzen R-BDₙₖ zugeordnet sind, ermittelt. Weiterhin wird bei dem Schritt 2.IV ermittelt, ob die Abweichung AB einen vorbestimmten Schwellwert SW erreicht oder überschreitet. Für den Fall, dass dies der Fall ist, was in FIG 2 mit "j" gekennzeichnet ist, wird zu dem Schritt 2.V übergegangen, bei dem die Resistenz-Ermittlungsfunktion modifiziert wird. Anschließend wird zu dem Schritt 2.II zurückgekehrt und es wird die modifizierte Resistenz-Ermittlungsfunktion REF₁ auf die Referenz-Bilddatensätze R-BDₙₖ angewandt. Die Schritte 2.II bis 2.V werden iterativ solange durchgeführt, bis die bei dem Schritt 2.IV ermittelte Abweichung AB einen vorbestimmten Schwellwert unterschreitet, was in FIG 2 mit "n" gekennzeichnet ist. Schließlich wird bei dem Schritt 2.VI die zuletzt genutzte Resistenz-Ermittlungsfunktion REFₘ als Resistenz-Ermittlungsfunktion REF für das in FIG 1 veranschaulichte Verfahren zum Überwachen von medizinischen Parametern in einem Kollektiv festgelegt.

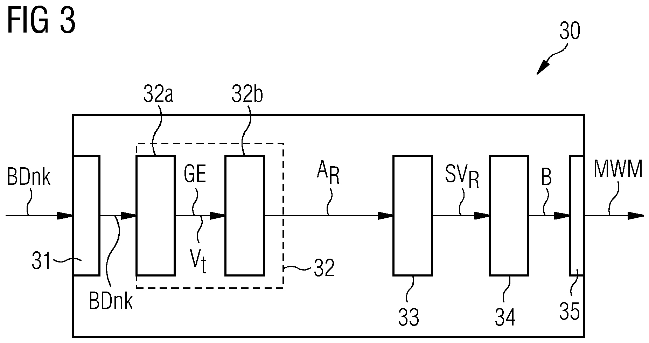

In FIG 3 ist ein Blockdiagramm gezeigt, mit dem eine Überwachungseinrichtung 30 gemäß einem Ausführungsbeispiel der Erfindung dargestellt wird. Die Überwachungseinrichtung 30 umfasst eine Eingangsschnittstelle 31, die dazu eingerichtet ist, Bilddatensätze BDₙₖ einer Mehrzahl von Mitgliedern Pₙ eines Kollektivs, beispielsweise von einer zentralen Datenbank zu empfangen. Teil der Überwachungseinrichtung 30 ist auch eine Klassifizierungseinheit 32 zum automatisierten Klassifizieren der Mitglieder Pₙ des Kollektivs auf Basis der erfassten Bilddatensätze BDₙₖ. Die Klassifizierungseinheit 32 umfasst eine Parameter-Ermittlungseinheit 32a, welche auf Basis der empfangenen Bilddatensätze BDₙₖ medizinische Parameter GE, Vₜ ermittelt. Die medizinischen Parameter GE, Vₜ können zum Beispiel die bereits im Zusammenhang mit FIG 1 und 2 genannten Biomarker, d.h. beispielsweise Informationen GE bezüglich der Fläche eines Entzündungsbereichs oder Informationen über den Verlauf Vₜ des dynamischen Verhaltens eines solchen Entzündungsbereichs umfassen. Teil der Klassifizierungseinheit 32 ist auch eine Klassenzuordnungseinheit 32b, welche auf Basis der ermittelten medizinischen Parameter GE, Vₜ eine Zuordnung der Mitglieder des Kollektivs zu einzelnen Klassen vornimmt. Einzelne Klassen können, wie bereits beschrieben, unterschiedlichen Werten oder Wertebereichen von Klassifizierungsparametern zugeordnet sein.

In dem in FIG 1 und FIG 2 beschriebenen Fall können einzelne Patienten Pₙ dem Klassifizierungsparameter A_{R} = 1 oder A_{R} = 0 zugeordnet werden. Wobei ersteres bedeutet, dass der Patient eine Resistenz aufweist und letzteres bedeutet, dass der Patient keine Resistenz aufweist. Teil der Überwachungseinrichtung 30 ist auch eine Verteilungsermittlungseinheit 33 zum automatisierten Ermitteln einer statistischen Verteilung SV_{R} der klassifizierten Mitglieder Pₙ des Kollektivs auf Basis der ermittelten Klassifizierungen A_{R}. Auf Basis der ermittelten statistischen Verteilung SV_{R} wird weiterhin von einer Vergleichseinheit 34 ein automatisierter Vergleich einer statistischen Referenzverteilung mit der ermittelten statistischen Verteilung SV_{R} durchgeführt und auf Basis des Vergleichs werden geographische Gebiete B ermittelt, welche bestimmten medizinisch wirksamen Maßnahmen MWM unterworfen werden sollen. Mit Hilfe einer Anweisungsausgabeeinheit 35 werden Anweisungen zu den medizinisch wirksamen Maßnahmen MWM an in den ermittelten geographischen Gebieten B liegende medizinische Betreuungseinrichtungen übermittelt.

Es wird abschließend noch einmal darauf hingewiesen, dass es sich bei den vorbeschriebenen Verfahren und Vorrichtungen lediglich um bevorzugte Ausführungsbeispiele der Erfindung handelt und dass die Erfindung vom Fachmann variiert werden kann, ohne den Bereich der Erfindung zu verlassen, soweit er durch die Ansprüche vorgegeben ist. So wurden das Verfahren und die Überwachungseinrichtung in erster Linie in Bezug auf eine geographische Verbreitung einer Resistenz gegen Antibiotika beschrieben. Die Erfindung ist jedoch nicht auf diese konkrete Anwendung beschränkt, sondern die Erfindung kann auch grundsätzlich auf eine Vielzahl unterschiedlicher, in großen Kollektiven auftretende medizinische Fragestellungen angewandt werden. Es wird der Vollständigkeit halber auch darauf hingewiesen, dass die Verwendung der unbestimmten Artikel "ein" bzw. "eine" nicht ausschließt, dass die betreffenden Merkmale auch mehrfach vorhanden sein können. Ebenso schließt der Begriff "Einheit" nicht aus, dass diese aus mehreren Komponenten besteht, die gegebenenfalls auch räumlich verteilt sein können.

## Patentansprüche

1. Verfahren zum Überwachen des Gesundheitsstatus eines Kollektivs, aufweisend die Schritte:
- Erfassen von medizinischen Bilddatensätzen (BDₙₖ) einer Mehrzahl von Mitgliedern (Pₙ) des Kollektivs,
- automatisiertes Klassifizieren der Mitglieder (Pₙ) des Kollektivs auf Basis der erfassten medizinischen Bilddatensätze (BDₙₖ) nach einem medizinisch relevanten Kriterium,
- automatisiertes Ermitteln einer statistischen Verteilung (SV_{R}) der klassifizierten Mitglieder des Kollektivs,
- automatisiertes Vergleichen einer statistischen Referenz-Verteilung mit der ermittelten statistischen Verteilung (SV_{R}).

2. Verfahren nach Anspruch 1, wobei bei dem Klassifizierungsschritt
- den einzelnen Mitgliedern (Pₙ) zugeordnete medizinische Parameter (GE, Vₜ) auf Basis der erfassten Bilddatensätze (BDₙ) ermittelt werden und
- das automatisierte Klassifizieren der Mitglieder des Kollektivs auf Basis der ermittelten medizinischen Parameter (GE, Vₜ) erfolgt.

3. Verfahren nach Anspruch 1 oder 2, wobei Steuermaßnahmen (MWM) zur Beeinflussung des Gesundheitsstatus zumindest eines Teils des Kollektivs auf Basis eines Ergebnisses des Vergleichs vorzugsweise automatisiert eingeleitet werden.

4. Verfahren nach einem der vorstehenden Ansprüche, wobei das automatisierte Klassifizieren auf Basis von Zeitreihen von Bilddatensätzen (BDₙₖ) erfolgt.

5. Verfahren nach einem der vorstehenden Ansprüche, wobei das automatisierte Klassifizieren der Mitglieder (Pₙ) des Kollektivs durch Anwendung eines trainingsbasierten Klassifizierungsverfahrens (REF) erfolgt.

6. Verfahren nach Anspruch 5, wobei das trainingsbasierte Klassifizierungsverfahren (REF) durch folgende Schritte trainiert wird:
- Anwenden eines initialen Klassifizierungsverfahrens (REF₀) auf eine Mehrzahl von Bilddatensätzen (R-BDₙₖ) mit vorbekannter Klassifizierung der den Bilddatensätzen (R-BDₙₖ) zugeordneten Mitgliedern (R-Pₙ) eines Referenz-Kollektivs,
- Vergleichen des Klassifizierungsergebnisses (A_{Rn}) mit einem vorbekannten Klassifizierungsergebnis (R-A_{Rn}) der Mehrzahl von Bilddatensätzen (R-BDₙₖ),
- Anpassen des initialen Klassifizierungsverfahrens (REF₀) auf Basis des Vergleichs,
- Wiederholen der vorgenannten Schritte mit dem angepassten Klassifizierungsverfahren (REFₘ), bis ein Qualitätskriterium für das angepasste Klassifizierungsverfahren erreicht ist,
- Festlegen des angepassten Klassifizierungsverfahrens (REFₘ) als anzuwendendes trainingsbasiertes Klassifizierungsverfahren (REF) für den Fall, dass das Qualitätskriterium für das angepasste Klassifizierungsverfahren (REFₘ) erreicht ist.

7. Verfahren nach Anspruch 5 oder 6, wobei das trainingsbasierte Klassifizierungsverfahren (REF) mit Hilfe eines maschinellen Lernverfahrens, vorzugsweise unter Anwendung eines künstlichen neuronalen Netzes, trainiert wird.

8. Verfahren nach einem der Ansprüche 2 bis 7, wobei die medizinischen Parameter Biomarker umfassen.

9. Verfahren nach einem der vorstehenden Ansprüche, wobei die statistische Verteilung (SV_{R}) der klassifizierten Mitglieder (Pₙ) des Kollektivs eine geographische statistische Verteilung umfasst.

10. Verfahren nach einem der vorstehenden Ansprüche, wobei das automatisierte Klassifizieren zusätzlich zu den erfassten Bilddatensätzen (BDₙₖ) auf Basis von den einzelnen Bilddatensätzen zugeordneten Zusatzinformationen erfolgt.

11. Verfahren nach Anspruch 10, wobei die Zusatzinformationen einen der folgenden Informationstypen umfassen:
- Diagnosedaten,
- Laborberichte,
- Datum und Zeitpunkt der Bildaufnahme der Bilddatensätze.

12. Verfahren nach einem der vorstehenden Ansprüche, wobei auf Basis des Vergleichs eine Prävalenz des Kollektivs bezüglich eine vorbestimmten Krankheit oder ein Ansprechen des Kollektivs auf ein Medikament ermittelt wird.

13. Überwachungseinrichtung (30), aufweisend:
- eine Eingangsschnittstelle (31) zum Erfassen von Bilddatensätzen (BDₙₖ) einer Mehrzahl von Mitgliedern (Pₙ) eines Kollektivs,
- eine Klassifizierungseinheit (32) zum automatisierten Klassifizieren der Mitglieder (Pₙ) des Kollektivs auf Basis der erfassten Bilddatensätze (BDₙₖ) nach einem medizinisch relevanten Kriterium,
- eine Verteilungsermittlungseinheit (33) zum automatisierten Ermitteln einer statistischen Verteilung (SV_{R}) der klassifizierten Mitglieder (Pₙ) des Kollektivs,
- eine Vergleichseinheit (34) zum automatisierten Vergleichen einer statistischen Referenzverteilung mit der ermittelten statistischen Verteilung (SV_{R}).

14. Computerprogrammprodukt mit einem Computerprogramm, welches direkt in eine Speichereinheit einer Überwachungseinrichtung (30) ladbar ist, mit Programmabschnitten, um alle Schritte des Verfahrens nach einem der Ansprüche 1 bis 12 auszuführen, wenn das Computerprogramm in der Überwachungseinrichtung (30) ausgeführt wird.

15. Computerlesbares Medium, auf welchem von einer Rechnereinheit ausführbare Programmabschnitte gespeichert sind, um alle Schritte des Verfahrens nach einem der Ansprüche 1 bis 12 auszuführen, wenn die Programmabschnitte von der Rechnereinheit ausgeführt werden.
